# EUROPEAN PATENT SPECIFICATION

(11) **EP 3 968 880 B1**
(45) Date of publication and mention of the grant of the patent: **22.03.2023**
(21) Application number: 19805791.1
(22) Date of filing: 30.09.2019
(51) Int. Cl.: A61B 17/72, A61B 17/00

(54) **AUTONOMOUS CONTROL AND LENGTHENING SYSTEM FOR TUMOR PROSTHESIS**
AUTONOMES STEUER- UND VERLÄNGERUNGSSYSTEM FÜR EINE TUMORPROTHESE
SYSTÈME DE COMMANDE ET D'ALLONGEMENT AUTONOME POUR PROTHÈSE TUMORALE

(30) Priority: 14.05.2019 TR 201907277
(43) Date of publication of application: 23.03.2022
(73) Proprietor: Yildiz Teknik Üniversitesi, 34220 Istanbul (TR)
(72) Inventor: KOCAOGLU, Sitki, 39100 Kirklareli (TR); AKDOGAN, Erhan, 34349 Besiktas/Istanbul (TR)
(74) Representative: Sevinç, Erkan
(86) International application number: PCT/TR2019/050811
(87) International publication number: WO 2020/231359

(56) References cited:
- CN-A- 105 662 663
- US-A1- 2018 317 980
- VERKERKE ET AL: "An extendable modular endoprosthetic system for bone tumour management in the leg", JOURNAL OF BIOMEDICAL ENGINEERING, BUTTERWORTH, GUILDFORD, GB, vol. 12, no. 2, 1 March 1990 (1990-03-01), pages 91-96, XP022444480, ISSN: 0141-5425, DOI: 10.1016/0141-5425(90)90126-8
- ANDERSON M ET AL: "Growth and predictions of growth in the lower extremities", J BONE JOINT SURG., vol. 45A, 1 January 1963 (1963-01-01), pages 1-14, XP002797574,

## Description

### Technical Field of the Invention

The present invention relates to autonomous extendable systems for tumor prostheses, and to a system including a wearable bone length measurement sensor, as defined in the claims.

### Background of the Invention

Currently, bone cancer patients who use extendable tumor prostheses need to often visit clinical facilities for determination of limb length differences and for extension in the case where it is necessary, as long as their age-related growth continues. This fact brings along several problems. These problems include inconveniences in daily life of patients along with increment in work load of the physicians; the patient being exposed to radiation at each measurement; large scale application of extension (at greater step lengths) due to that the extension is applied periodically (i.e. intermittently, or at intervals), and accordingly decreasing the patient comfort and increasing the work load of the physicians. State of the art tumor prostheses are extended by rotation of a motor inside the prosthesis by an external electromagnetic field. Determination of a required amount of extension is performed by frequent visits of the patient to a clinical facility and by specifying the limb length difference due to the time past from a previous visit. In the case where a great extent of limb length difference has occured, the extension procedure is performed again in a clinical facility by a physician.

The document CN 10 566 2663 relates to the present technical field. With the system disclosed in said document, the determination of when and to which extent the femur of the patient is to be extended, relies on statistical and estimation-based data which produce rather subjective and only approximate results: e.g. the body weight of the patient, Chinese percentile tables, keeping track and perception by the patient's family, age of implantation, or the number of steps taken by the patient. No information is given on how to recharge the battery which is expected to serve for around 8 years.

With the current systems the difference between limb length cannot be measured autonomously, the daily life of patients being adversely affected because of the necessity to periodically visit medical facilities, high work load of physicians, the patients being frequently subjected to radiation at measurements, and deterioration of patients' life comfort due to requirement of extension in high extents because of that the extension process is applied intermittently.

### Objects of the invention

The primary object of the present invention is to eliminate the above-mentioned shortcomings in the present state of the art.

### Summary of the Invention

The present invention proposes a bone extension system for tumor prostheses, comprising a prosthesis which includes an internal battery suitable for being charged without necessitating wiring; the system further comprises an extendable mechanism connected to the prosthesis; said extendable mechanism being arranged to, when in use, based on the length of the healthy limb, bring the length of a limb provided with said mechanism to a length which is substantially equal to that of the healthy limb.

### Brief Explanation of the Figures

The figures brief explanation of which is herewith provided are solely intended for providing a better understanding of the present invention and are as such not intended to define the scope of protection or the context in which said scope is to be interpreted in the absence of the description.

The US patent application US 2018/317980 A1, scientific research articles of Verkerke et al. (DOI: 10.1016/0141-5425(90)90126-8) and Anderson Met al. ("Growth and predictions of growth in the lower extremities", J BONE JOINT SURG., vol. 45A, 01.01.1963) and Chinese patent application CN 105 662 663 A relate to the technical field of the present application.
Fig.1 is a diagram showing the functioning between a preferred set of features in an exemplary embodiment of a system according to the present invention.
Fig.2 shows a section view of an exemplary embodiment of a system according to the present invention.
Fig.3 shows an exemplary use of an exemplary embodiment of the system according to the present invention, on an experimental setup simulating a patient.

### Detailed Description of the Invention

Referring to the figures outlined before, the present invention is disclosed below in details. The reference numbers used in the figures are listed as:
- 1.: extendable mechanism
- 11.: knee joint
- 12.: outer tube
- 13.: inner tube
- 2.: combination of motor drive, motor, spindle drive and encoder
- 21.: motor drive / reductor
- 22.: motor
- 23.: spindle drive (rotatable driving member)
- 24.: encoder
- 3.: internal control unit
- 4.: external control unit
- 5.: external sensor (e.g. wearable sensor)
- 6.: charging unit
- 7.: (internal-) battery
- 8.: (user)
- 100.: system
- 101.: electrical energy
- 102.: battery voltage
- 103, 104.: information - command
- 105.: extension signal
- 106.: length change
- 107.: healthy limb length
- 108.: data signal (prosthesis length)
- 201.: healthy limb
- 202.: prosthesis
- 203.: healthy limb
- 204.: extending module

For eliminating the shortcomings mentioned in the above "background" section, an extendible, motor-driven, intelligent tumor prosthesis (system, 100) is developed. An important difference of the system proposed in the present invention from those mentioned in the background section, is that the system according to the present invention is autonomous.

In the system (100) according to the present invention, a battery (7) is employed. Said battery (7) is an internal battery (battery, 7) which can be for example disposed inside a tumor prosthesis (prosthesis, 202) in the system (100). The battery (7) is suitable for being charged by an external energy source without necessitating to be brought to mechanical contact therewith. Said battery (7) is arranged for being charged in a "wireless" fashion.

Further, an external sensor (5) unit is proposed within the context of the present invention, for measuring length of a healthy limb. Said external sensor (5) unit is preferably arranged to be wearable. The system (100) according to the present invention is arranged for a machine-learning-based estimation of a healthy limb length and a patient posture situation, based on measurements provided by the external sensor (5).

A patient with a prosthesis (202) on a limb, can wear the external (e.g. wearable) sensor (5) unit on another, healthy limb (201) (e.g. in the case where a femur is provided with a prosthesis, onto the leg of the other femur); and enables the tracking of a "length of the healthy limb" data obtained therefrom. Said length of the healthy limb data is communicated to an "external control unit" (4).

The system (100) further comprises an extendable mechanism (1) connected to the prosthesis (202). Said extendable mechanism (1) is suitable for being extended to bring the length of the limb provided with the prosthesis, based on the length of the healthy limb, to a value substantially equal to the length of the healthy limb. The extendable mechanism (1) has a telescopic structure. For the sake of being lightweight, the extendible mechanism (1) comprises two coaxially arranged tubes (pipes) (12 and 13) which are slidable relative to each other along an extension axis.

The prosthesis (202) is provided with a sensor which is suitable for measuring the length of a limb (108) which is provided with said prosthesis. The sensor is arranged to determine momentary positions of said two tubes (12 and 13) relative to each other. A data signal (108) is based on said relative positions, and then is communicated to an "internal control unit" (3). The length of the limb provided with the prosthesis can be thus measured by calculation based on the magnitude of said data signal (108) which is based on said relative positions.

The prosthesis (202) is be provided with a feature arranged to make a comparison between the healthy limb length (107) and the lengh (108) of the limb provided with the prosthesis, and to then determine a "difference data" showing the difference between the healthy limb length and the length (108) of the limb provided with the prosthesis (i.e. how much the length (108) of the limb provided with the prosthesis should be altered -e.g. extended-). Hence, it can be determined how much the length (108) of the limb provided with the prosthesis is to be (ultimately) altered.

The prosthesis (202) is provided with an adjusting means for arranging the length of the extendable mechanism (1), to bring the length (108) of the limb provided with the prosthesis to a value equal to or closer to the healthy limb length (107), based on data related to the healthy limb length (107) and the lengh (108) of the limb provided with the prosthesis, e.g. based on the difference data. Said adjusting means can include a motor drive (21), a motor (22), a spindle drive (23) (rotatable driving means) and an encoder (24) altogether.

Thus the system (100) can alter (e.g. lengthen) the length of the prosthesis (20) based on the healthy limb length (107), without necessitating a visit to a medical facility. Furthermore, this procedure can be performed much more often with regard to the intervals applied in the present state of the art; and thus the differences in the prosthesis (202) lengths can be very small when compared with those encountered in the present state of the art, at each time when the lengh (108) of the limb provided with the prosthesis is to be updated i.e. altered. The number of necessary visit to medical facilities is thus minimized, the extent of discomfort related to the pain to which the patient is subjected to is decreased, the patient comfort is enhanced, the lengthening procedure is facilitated, and the work load of the physicians is minimized.

The system (100) can be arranged to determine a time for altering the lengh (108) of the limb provided with the prosthesis (e.g. based on the length (107, 108) data mentioned above or on the difference data), and to generate a warning signal accordingly. Said warning signal can be communicated over a visual and/or audial interface. The system (100) can be arranged for triggering of the function of altering the lengh (108) of the limb provided with the prosthesis, at a time preferred by the patient or at a time which suits to the patient, after said generation of the warning signal.

For better understanding of the preferred embodiments according to the present invention, the following example is provided:

### EXAMPLE:

In an exemplary embodiment, the system (100) according to the present invention can include the following features:
- The extendable mechanism (1) enables a longitudinal extension of the prosthesis (202). The extendable mechanism includes coaxial tubes (12 and 13).
- Combination of the motor drive (21), motor (22), spindle drive (23) and encoder (24):
   enables the longitudinal extension of the extendable mechanism (1) at a stride length and with a sufficient extent of power, and the measurement of a momentary length. The known, state of the art extendable prostheses include a magnetic rotor and screw-nut mechanisms communicating the motion of said rotor to the extendable mechanism, and such mechanisms can be considered as a primitive version of the spindle drive unit. Yet, the measurement of the lengthening from the inside of the tumor prosthesis by the encoder (24) has not ever been put into practise with known interbody implants (including known tumor prostheses) prior to the present invention.
- The internal control unit (3) is the main controlling means of the system (100). It makes the system (100) function, by using information collected from other features of the system, and by generating commands accordingly. For the first time, a central controlling unit is established in a tumor prosthesis.
- The external control unit (4) enables communication between the system (100) and the user, and between the system (100) and the wearable sensor (5). The external control unit determines whether an extension is necessary, and communicates an extension command to the internal control unit (3).
- The wearable sensor (5) uses sensors therein and thereby enables the measuring of the healthy limb length (107). Hence, the measurement of a part inside the body (e.g. the length of a femur) can be achieved using a sensor.
- Charging unit enables the charging of the battery (7) (which in use remains within the patient's body), by a charge control module and charge coils inside said charging unit.

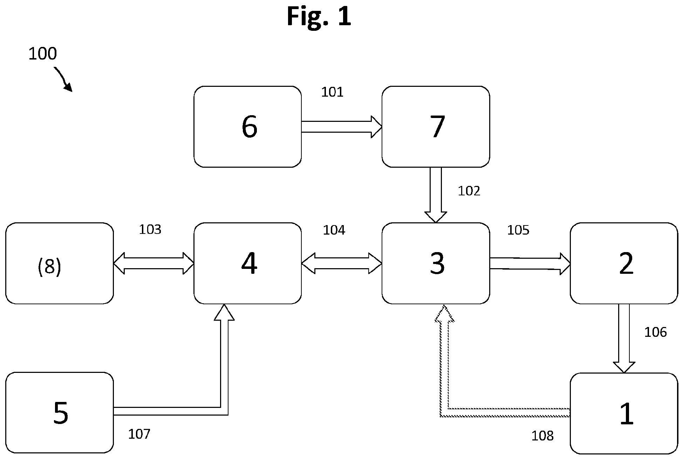

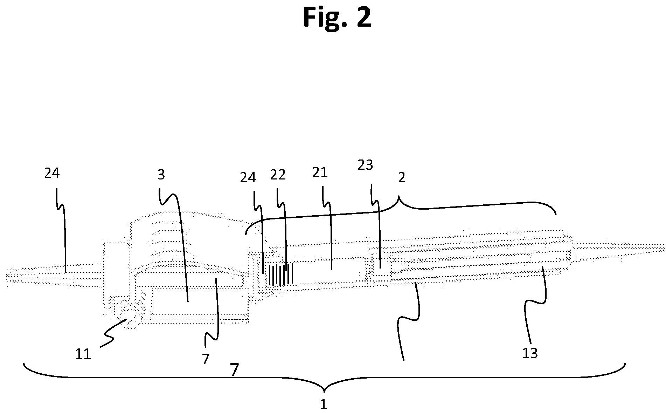

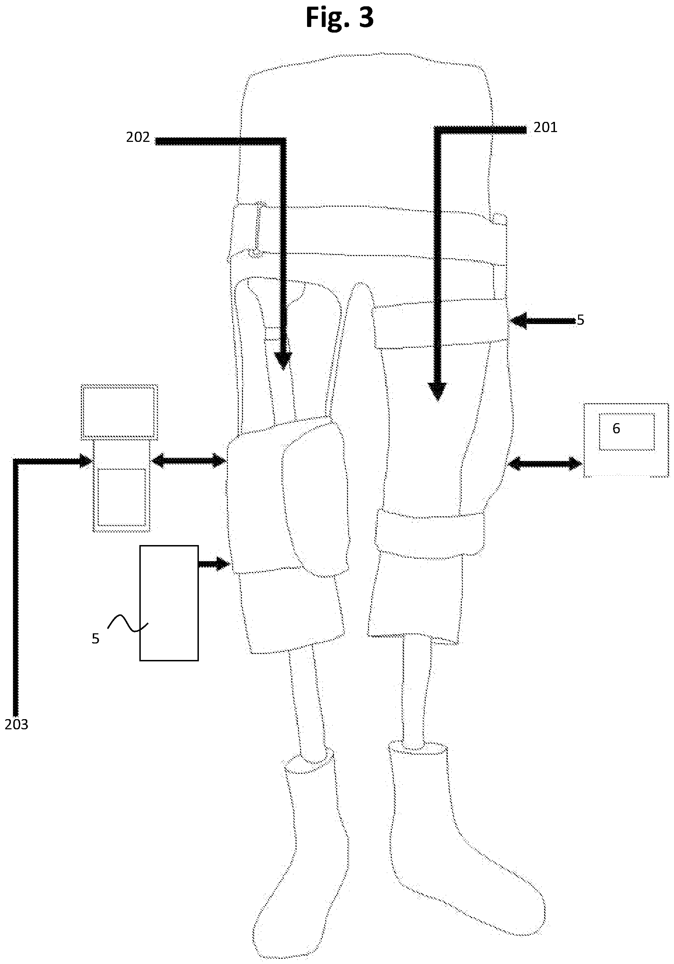

The diagram shown in the Fig.1 is hereby referred to for better understanding said exemplary system according to the present invention. The units numbered as 1, 2, 3 and 4 in the diagram and preferable sub-constituents thereof are shown in the Fig.2; and the units numbered as 5, 6 and 7 are represented on an experimental setup shown in the Fig.3. The other constituents shown in the Fig.3 are used as test-related and experimental means, and are not to be considered as limiting to the scope of protection.
1) Extendible mechanism (1): The extendible mechanism includes an artificial knee joint (11) and at least 2 tubes (12 and 13) which are arranged coaxially. One of the tubes (12 and 13) is connectable to the femur via a stem, and a lower portion of the artificial knee joint (11) is connectable to the tibia via a corresponding stem. Once the motor (22) is driven, the two tubes (12 and 13) move relative to each other and distanced away from each other by the spindle drive (23). Thereby the prosthesis (202) is lengthened.
2) The combination of motor drive (21), motor (22), spindle drive (23) and encoder (24): A combination of motor (22) (e.g. a 4.5 V brushless DC motor with a nominal torque of 3.33 mNm) and spindle drive (23) (e.g. having a conversion ratio of 850:1) is used in the system. A motor drive (21) is introduced to drive the motor (22). Position information of the motor (22) is taken from a 3-channel incremental encoder (24) (e.g. with 3-channels) introduced to the combination. With the combination, a force of 378 N and a low lengthening rate (with a maximum of 0.22 mm/min) can be achieved at the lengthening process.
3) Internal control unit (3): With the sensors of the internal control unit (3) which can be placed into the knee joint of the prosthesis (202), prosthesis (202)-related physical values (e.g. an extent of lengthening, temperature, charge level of the internal battery (7) and information on patient posture situation) can be measured. The internal control unit communicates the information obtained from the sensors (such as an increment of an inner temperature, a decrement of the internal battery charge level, patient posture situation) with the external control unit (4). When the lengthening process is to be applied, the internal control unit receives a command from the external control unit (4) and controls via sensors whether the conditions are suitable for lengthening. During the lengthening process, the internal control unit is the unit which controls the motor drive (21) and supervises the lengthening process. The internal temperature of the prosthesis (202) can be regularly followed by a temperature sensor which functions under the internal control unit (3). When the temperature rises to undesirable values, the internal control unit (3) can get the constituents of the prosthesis to a passive mode and aborts the lengthening process.
4) External control unit (4): The external control unit which can be in the form of an HMI panel, is arranged to enable the communication of the patient as user with the system. The external control unit can evaluate information from the internal control unit (3) and when necessary, inform the patient accordingly. The external control unit can compare the healthy limb length information obtained via the wearable sensor (5) with the length of the limb provided with the prosthesis. Thus a necessity of lengthening is determined and communicated to the patient as user, and to the internal control unit (3); provides a lengthening command if necessary. The external control unit starts and stops charging of the internal batteries (7).
5) External (e.g. wearable) sensor (5): The patient as user can connect the wearable sensor (5) to his healthy limb from outside. The wearable sensor (5) can have a flexible structure and can be stretched as long as the length of the healthy limb when connected to said limb. Transmitters (e.g. passive RFID tags) which can be located at lower and upper ends of a femur of a healthy limb of the patient, and sensors located in the wearable sensor (5) unit can be aligned with each other and can get communicated with each other. Whether said alignment is performed properly, can be supervised by the external control unit (4) and the patient as user can be guided for arranging a proper alignment. Once a proper alignment is achieved, the length of the wearable sensor (5) can be measured by a length measurement sensor (e.g. a linear potentiometer, an infrared sensor, an ultrasound sensor or a laser sensor) inside the wearable sensor (5). The length of the wearable sensor (5) is an indicator of the healthy limb length (107). This value of length of the wearable sensor can be communicated to the internal control unit (3) by the external control unit (4). Limb length differences arising due to the growth of the healthy limb can be sensed by the wearable sensor (5). When such limb length difference arises, the system (100) starts the lengthening procedure by guiding the patient as user.
6) Charging unit (6): The energy of the prosthesis (202) is supplied by the battery (7) which can be located inside the artificial knee joint (11). Battery charging level information can be regularly measured by the internal control unit (3); and a warning signal can be communicated to the external control unit (4) when the battery charging level drops below a pre-determined threshold level for commencement of charging. Thus the charging unit provides charging of the battery by the patient as user. A battery charge control unit can be employed for provision of security and for protection of the battery (7). Hence, when the battery voltage drops to a value below a threshold level, a discharging operation is commenced, and when the battery voltage raises to a value above said threshold level, the charging operation is finalized.
7) Internal battery (7): The internal battery (e.g. Li-ion) can be placed inside the artificial knee joint (11) for providing energy to the prosthesis (202).
8) User (8): The user can be a patient having the tumor prosthesis implanted to his body.

The system (101) can measure a healthy limb length (107) of the patient, and can autonomously make a decision to lengthening upon comparing said healthy limb length with the length (108) of a limb provided with the prosthesis. The prosthesis (202) can be arranged to be suitable for provide measurements such as an extent of lengthening, temperature, internal battery charge level and prosthesis position information, when an internal control unit (3) suitable for being placed inside e.g. a knee joint is in operation.

The external sensor (5) can be arranged as a wearable sensor. The external sensor is suitable for being approximated to a healthy limb (201) by a user (8) (e.g. a patient), e.g. by tying in the case where the external sensor is wearable. By communication of sensors corresponding to lower and upper distal ends of a healthy limb (201) (e.g. femur) with the wearable sensor (5) unit, the healthy limb length (107) can be determined and then communicated to the internal control unit (3) via the external control unit (4). If necessary and if the conditions are suitable, the lengthening operation can be commenced by informing and guiding the patient as user. It is preferred that a daily lengthening step length of the extendable mechanism (1) is limited to 1 mm, to allow healing of a soft tissue at a related treatment zone and to allow entrance of muscles to a relaxation phase. Thus, a comfortable and safe limb lengthening can be achieved without necessitating to visit a clinical facility.

An exemplary use of the system (100) according to the present invention can include the following features:
a) The prosthesis (202) can be implanted into a bone of a patient. For instance, a tumored part of a femur of a pediatric patient can be surgically removed along with the corresponding knee joint and growth plate, and replaced with the prosthesis of the system according to the present invention.
b) A measuring means can be employed for following the healthy limb length (107) which pairs the limb provided with the prosthesis. For instance, transmitters can be aligned at two distal ends of a bone (in this example: femur) at the healthy limb, for measuring a distance between said distal ends. This procedure can be conducted simultaneous to the surgical operation at the step (a).
c) A healthy limb length data can be obtained via a sensor which is arranged to externally sense a momentary situation of the measuring means which is mentioned in the step (b). For instance, periodically, the patient as user can externally tie a wearable, external sensor (5) onto his healthy limb, being guided step-by-step by an external control unit. Once the sensors and transmitters are aligned with each other, e.g. by approximately being faced with each other, the healthy limb length (107) data gets generated, e.g. the length sensor communicates a measured value to an external control unit (4).
d) An internal control unit (3) can be arranged to keep a prosthesis (202) momentary length information or a length (108) information of a limb provided with the prosthesis in a memory thereof, using an encoder (24) which is possibly provided in the structure of the prosthesis (202). Such information can be communicated to the external control unit (4).
e) The external control unit (4) can be arranged to specify a difference between the healthy limb length (107) and the lengh (108) of the limb provided with the prosthesis. The system (100) can be arranged to guide the patient by commands and thereby prepare the patient for a lengthening procedure, once the difference is specified to be above a pre-determined value, e.g. by the structure of the external control unit (4) being arranged accordingly.
f) The external control unit (4) can be arranged to, by receiving prosthesis sensor information (e.g. temperature, patient posture situation, internal battery charge level) from the internal control unit (3), commence/trigger a lengthening procedure in the case where the conditions are suitable.

Preferable embodiments of the system (100) according to the present invention can be arranged to provide one or more of the following advantageous technical effects:
- Provision of measurement of a length (107) of a bone in a healthy limb of a patient (e.g. the length of the femur in a healthy leg of the patient).
- Provision of measurement of a momentary length (108) of the prosthesis without bringing discomfort to the patient.
- The system can be equipped for measuring the posture situation of the patient via an AHRS sensor and machine learning (e.g. by including an accordingly pre-programmed controller), in order to avoid that a lengthening procedure is conducted when the patient is standing.
- The system can centrally control and decide thanks to the internal control unit (3) and external control unit (4), thus can function in an autonomous fashion.
- The prosthesis (202) can include a temperature measuring means for measuring an internal temperature of said prosthesis, for protecting the same from dangerous temperature levels.
- The internal battery (7) can be provided with a sensor, for measuring momentary charge level thereof.
- The internal battery (7) can be arranged to allow (externally, or remotely) recharging in a wireless fashion.

## Claims

1. A bone lengthening system (100) for tumor prostheses, comprising a prosthesis (202) which includes an internal battery (7) arranged for wireless charging; the system (100) further comprises an extendable mechanism (1)
which is connected to the prosthesis (202) and
which is arranged to be lengthened via an adjusting means for, when in use, altering a length (108) of a limb provided with the prosthesis (202) based on a healthy limb length (107) data;
said extendable mechanism (1) has a telescopic structure by including coaxially arranged two tubes (12 and 13) which are slidable relative to each other along an extension axis; and
the prosthesis (202) is provided with a sensor which is suitable for measuring the length (108) of the limb which is provided with said prosthesis; the sensor being arranged to determine momentary positions of said two tubes (12 and 13) relative to each other and generate a data signal based on said relative positions corresponding to the length (108) of the limb provided with the prosthesis (202);
**characterized in that**
the prosthesis (202) includes an internal control unit (3) arranged to receive communication of the data signal corresponding to the length (108) of the limb which is provided with said prosthesis; and the system further comprises an external control unit (4) and an external sensor (5) for being externally arranged onto a healthy limb for measuring and tracking a healthy limb length (107) data to be communicated to the external control unit (4).

2. The system according to the claim 1, wherein the internal control unit (3) includes sensors for gathering information on one or more prosthesis (202) - related physical values selected from the list consisting of an extent of lengthening, a temperature, a battery charge level and preferably information on patient posture situation; and the internal control unit (3) is arranged to communicate the information on said one or more prosthesis (202) - related physical values to the external control unit (4); the external control unit (4) being arranged to communicate a command for altering the length (108) of the limb which is provided with said prosthesis, to the internal control unit (3).

3. The system according to any of the claims 1 or 2, wherein the external sensor (5) is arranged to, when approximated to a healthy limb (201), communicate with further sensors corresponding to lower and upper distal ends of the healthy limb (201) to determine the healthy limb length (107) data and then communicate said healthy limb length (107) data to the internal control unit (3) via the external control unit (4).

4. The susyem according to any of the claims 1 to 3, wherein the external control unit (4) is arranged to provide information and guidance to a user for a commencement of the altering of the length (108) of the limb provided with the prosthesis (202).

5. The system according to any of the claims 1 to 4, being arranged to determine a time for altering the lengh (108) of the limb provided with the prosthesis based on the length (107 and 108) data, and to generate a warning signal accordingly; preferably the system being further arranged to communicate the warning signal over a visual and/or audial interface.

6. The system according to the claim 5, being arranged to trigger the altering of the lengh (108) of the limb provided with the prosthesis (202) at a time after the generation of said warning signal.

7. The system according to any of the claims 1 to 6, wherein the extendible mechanism includes an artificial knee joint (11) and one of said tubes (12 or 13) is connectable to a femur, and a portion of the artificial knee joint (11) is connectable to a tibia; the adjusting means comprises a motor (22), a spindle drive (23) and an encoder (24); said motor (22) being arranged to move the two tubes (12 and 13) relative to each other via the spindle drive (23).

8. The system according to any of the claims 1 to 7, comprising a measuring means which include transmitters for being aligned at two distal ends of a bone at a healthy limb of a patient, for measuring a distance between said two distal ends by externally sensing a momentary situation of the measuring means by the external sensor (5), and for then generating the healthy limb length (107) data for being communicated to the external control unit (4).

9. The system according to any of the claims 1 to 8, wherein the external control unit (4) is arranged to specify a difference between the healthy limb length (107) and the lengh (108) of the limb provided with the prosthesis (202).

10. The system according to the claim 9, arranged to guide the patient by commands once the difference is specified to be above a pre-determined value.

11. The system according to any of the claims 1 to 10, wherein the internal battery is disposed inside the prosthesis (202).

## Patentansprüche

1. Knochenverlängerungssystem (100) für Tumorprothesen, aufweisend eine Prothese (202), die eine interne Batterie (7) beinhaltet, die für drahtloses Laden konfiguriert ist; wobei das System (100) ferner einen ausziehbaren Mechanismus (1) aufweist, der mit der Prothese (202) verbunden ist und der so konfiguriert ist, dass er über eine Einstelleinrichtung verlängert werden kann, um während der Benutzung eine Länge (108) einer mit der Prothese (202) versehenen Gliedmaße auf Basis von Daten über die Länge einer gesunden Gliedmaße (107) zu verändern;
wobei der ausziehbare Mechanismus (1) eine teleskopische Struktur aufweist, indem er zwei koaxial angeordnete Röhren (12 und 13) beinhaltet, die relativ zueinander entlang einer Verlängerungsachse schiebbar sind; und
wobei die Prothese (202) über einen Sensor verfügt, der geeignet ist, die Länge (108) der mit der Prothese (202) versehenen Gliedmaße zu messen; wobei der Sensor so konfiguriert ist, dass er momentane Positionen der zwei Röhren (12 und 13) relativ zueinander bestimmt und auf Basis der relativen Positionen ein Datensignal erzeugt, das der Länge (108) der mit der Prothese (202) versehenen Gliedmaße entspricht;
**dadurch gekennzeichnet, dass**
die Prothese (202) eine interne Steuereinheit (3) beinhaltet, die so konfiguriert ist, dass sie eine Übertragung des Datensignals, das der Länge (108) der mit der Prothese (202) versehenen Gliedmaße entspricht, empfängt; und wobei das System ferner eine externe Steuereinheit (4) und einen externen Sensor (5) aufweist, der extern an einer gesunden Gliedmaße angeordnet ist, um Daten über die Länge einer gesunden Gliedmaße (107) zu messen und zu überwachen, um sie an die externe Steuereinheit (4) zu übertragen.

2. System nach Anspruch 1, wobei die interne Steuereinheit (3) Sensoren zum Sammeln von Informationen über entsprechende physikalische Werte einer oder mehrerer Prothesen (202) beinhaltet, die aus der Liste ausgewählt sind, die aus einem Ausmaß der Verlängerung, einer Temperatur, einem Batterieladezustand und vorzugsweise Informationen über die Haltungssituation des Patienten besteht; und wobei die interne Steuereinheit (3) so konfiguriert ist, dass die die Informationen über die entsprechenden physikalischen Werte der einen oder der mehreren Prothesen (202) an die externe Steuereinheit (4) überträgt; wobei die externe Steuereinheit (4) so konfiguriert ist, dass sie einen Befehl zum Ändern der Länge (108) der Gliedmaße, die über die Prothese verfügt, an die interne Steuereinheit (3) überträgt.

3. System nach einem der Ansprüche 1 oder 2, wobei der externe Sensor (5) so konfiguriert ist, dass er, wenn er einer gesunden Gliedmaße (201) angenähert ist, mit weiteren Sensoren kommuniziert, die unteren und oberen distalen Enden der gesunden Gliedmaße (201) entsprechen, um die Daten über die Länge der gesunden Gliedmaße (107) zu bestimmen und dann diese Daten über die Länge der gesunden Gliedmaße (107) über die externe Steuereinheit (4) an die interne Steuereinheit (3) zu übertragen.

4. System nach einem der Ansprüche 1 bis 3, wobei die externe Steuereinheit (4) so konfiguriert ist, dass sie einem Benutzer Informationen und Anleitung für ein Beginnen des Änderns der Länge (108) der mit der Prothese (202) versehenen Gliedmaße bietet.

5. System nach einem der Ansprüche 1 bis 4, wobei das System so konfiguriert ist, dass es eine Zeit für das Ändern der Länge (108) der mit der Prothese (202) versehenen Gliedmaße auf Basis der Längendaten (107 und 108) bestimmt, und entsprechend ein Warnsignal erzeugt; wobei das System vorzugsweise ferner so konfiguriert ist, dass es das Warnsignal über eine visuelle und/oder akustische Schnittstelle überträgt.

6. System nach Anspruch 5, wobei das System so konfiguriert ist, dass es das Ändern der Länge (108) der mit der Prothese (202) versehenen Gliedmaße zu einer Zeit nach der Erzeugung des Warnsignals auslöst.

7. System nach einem der Ansprüche 1 bis 6, wobei der ausziehbare Mechanismus ein künstliches Kniegelenk (11) beinhaltet und eine der Röhren (12 oder 13) mit einem Oberschenkelknochen verbunden werden kann, und ein Abschnitt des künstlichen Kniegelenks (11) mit einem Schienbein verbunden werden kann; wobei die Einstelleinrichtung einen Motor (22), einen Spindeltrieb (23) und einen Kodierer (24) aufweist; wobei der Motor (22) so konfiguriert ist, dass er die zwei Röhren (12 und 13) über den Spindeltrieb (23) relativ zueinander bewegt.

8. System nach einem der Ansprüche 1 bis 7, aufweisend eine Messeinrichtung, die Sender beinhaltet, um an zwei distalen Enden eines Knochens bei einer gesunden Gliedmaße eines Patienten ausgerichtet zu sein, um eine Entfernung zwischen den zwei distalen Enden durch ein externes Detektieren einer momentanen Situation der Messeinrichtung durch den externen Sensor (5) zu messen, und dann die Daten über die Länge der gesunden Gliedmaße (107) zu erzeugen, um sie an die externe Steuereinheit (4) zu übertragen.

9. System nach einem der Ansprüche 1 bis 8, wobei die externe Steuereinheit (4) so konfiguriert ist, dass sie eine Differenz zwischen der Länge der gesunden Gliedmaße (107) und der Länge (108) der mit der Prothese (202) versehenen Gliedmaße spezifiziert.

10. System nach Anspruch 9, das so konfiguriert ist, dass es den Patienten durch Kommandos anleitet, sobald bestimmt wird, dass sich die Differenz über einem vorgegebenen Wert befindet.

11. System nach einem der Ansprüche 1 bis 10, wobei die interne Batterie sich im Inneren der Prothese (202) befindet.

## Revendications

1. - Système d'allongement d'os (100) pour prothèses tumorales, comprenant une prothèse (202) qui comprend une batterie interne (7) disposée pour une charge sans fil ; le système (100) comprend en outre un mécanisme extensible (1)
qui est relié à la prothèse (202) et
qui est disposé pour être allongé par l'intermédiaire d'un moyen d'ajustement pour, lors de l'utilisation, modifier une longueur (108) d'un membre pourvu de la prothèse (202) sur la base d'une donnée de longueur de membre sain (107) ; ledit mécanisme extensible (1) a une structure télescopique en comprenant deux tubes disposés coaxialement (12 et 13) qui sont aptes à coulisser l'un par rapport à l'autre le long d'un axe d'extension ; et
la prothèse (202) est pourvue d'un capteur qui est approprié pour mesurer la longueur (108) du membre qui est pourvu de ladite prothèse ; le capteur étant disposé pour déterminer des positions momentanées desdits deux tubes (12 et 13) l'un par rapport à l'autre et générer un signal de données sur la base desdites positions relatives correspondant à la longueur (108) du membre pourvu de la prothèse (202) ;
**caractérisé par le fait que**
la prothèse (202) comprend une unité de commande interne (3) disposée pour recevoir une communication du signal de données correspondant à la longueur (108) du membre qui est pourvu de ladite prothèse ; et le système comprend en outre une unité de commande externe (4) et un capteur externe (5) destiné à être disposé extérieurement sur un membre sain pour mesurer et suivre une donnée de longueur de membre sain (107) à communiquer à l'unité de commande externe (4).

2. - Système selon la revendication 1, dans lequel l'unité de commande interne (3) comprend des capteurs pour recueillir des informations sur une ou plusieurs valeurs physiques liées à une prothèse (202) choisies dans la liste consistant en une étendue d'allongement, une température, un niveau de charge de batterie et de préférence des informations sur une situation de posture de patient ; et l'unité de commande interne (3) est disposée pour communiquer les informations relatives à ladite une ou auxdites plusieurs valeurs physiques liées à une prothèse (202) à l'unité de commande externe (4), l'unité de commande externe (4) étant disposée pour communiquer une commande pour modifier la longueur (108) du membre qui est pourvu de ladite prothèse, à l'unité de commande interne (3) .

3. - Système selon l'une quelconque des revendications 1 ou 2, dans lequel le capteur externe (5) est disposé pour, lorsqu'il est approché d'un membre sain (201), communiquer avec d'autres capteurs correspondant aux extrémités distales inférieure et supérieure du membre sain (201) pour déterminer les données de longueur de membre sain (107) puis communiquer lesdites données de longueur de membre sain (107) à l'unité de commande interne (3) par l'intermédiaire de l'unité de commande externe (4).

4. - Système selon l'une quelconque des revendications 1 à 3, dans lequel l'unité de commande externe (4) est disposée pour fournir des informations et des instructions à un utilisateur pour un commencement de la modification de la longueur (108) du membre pourvu de la prothèse (202) .

5. - Système selon l'une quelconque des revendications 1 à 4, qui est disposé pour déterminer un moment pour modifier la longueur (108) du membre pourvu de la prothèse sur la base des données de longueur (107 et 108), et pour générer un signal d'avertissement en conséquence, de préférence le système étant en outre disposé pour communiquer le signal d'avertissement sur une interface visuelle et/ou auditive.

6. - Système selon la revendication 5, qui est disposé pour déclencher la modification de la longueur (108) du membre pourvu de la prothèse (202) à un moment après la génération dudit signal d'avertissement.

7. - Système selon l'une quelconque des revendications 1 à 6, dans lequel le mécanisme extensible comprend une articulation artificielle de genou (11) et l'un desdits tubes (12 ou 13) est apte à être relié à un fémur, et une partie de l'articulation artificielle de genou (11) est apte à être reliée à un tibia ; le moyen d'ajustement comprend un moteur (22), un entraînement à broche (23) et un codeur (24), ledit moteur (22) étant disposé pour déplacer les deux tubes (12 et 13) l'un par rapport à l'autre par l'intermédiaire de l'entraînement à broche (23).

8. - Système selon l'une quelconque des revendications 1 à 7, comprenant un moyen de mesure qui comprend des émetteurs destinés à être alignés à deux extrémités distales d'un os à un membre sain d'un patient, pour mesurer une distance entre lesdites deux extrémités distales par détection externe d'une situation momentanée du moyen de mesure par le capteur externe (5), et pour ensuite générer les données de longueur de membre sain (107) pour être communiquées à l'unité de commande externe (4) .

9. - Système selon l'une quelconque des revendications 1 à 8, dans lequel l'unité de commande externe (4) est disposée pour spécifier une différence entre la longueur de membre sain (107) et la longueur (108) du membre pourvu de la prothèse (202).

10. - Système selon la revendication 9, disposé pour guider le patient par des commandes une fois que la différence est spécifiée comme étant supérieure à une valeur prédéterminée.

11. - Système selon l'une quelconque des revendications 1 à 10, dans lequel la batterie interne est disposée à l'intérieur de la prothèse (202).
